(19) Europäisches Patentamt
European Patent Office
Office européen des brevets

(11) Publication number: **0 305 667**
**A2**

# (12) EUROPEAN PATENT APPLICATION

(21) Application number: **88109992.3**

(22) Date of filing: **23.06.88**

(51) Int. Cl.[4]: **C07D 471/04 , //(C07D471/04, 239:00,221:00)**

(30) Priority: **03.09.87 US 92792**

(43) Date of publication of application: **08.03.89 Bulletin 89/10**

(84) Designated Contracting States: **AT BE CH DE ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Harris Boschelli, Diane**
**Jean Marie Gardens Apt. 9E**
**Nanuet New York 10954(US)**
Inventor: **Powell, Dennis William**
**50 Maple Moor Lane**
**Peekskill New York 10566(US)**

(74) Representative: **Wächtershäuser, Günter, Dr. et al**
**Tal 29**
**D-8000 München 2(DE)**

(54) **Improved process for producing 5,10-dideaza-(5,6,7,8)-tetrahydrofolic acid.**

(57) An improved process for producing the compound 5,10-dideazatetrahydrofolic acid.

EP 0 305 667 A2

# IMPROVED PROCESS FOR PRODUCING 5,10-DIDEAZATETRAHYDROFOLIC ACID

This invention relates to an improved process for producing the compound 5,10-dideazatetrahydrofolic acid, also referred to herein as DDA-THF, which significantly reduces the number of steps required to produce said compound and provides a substantial increase in yield as compared to prior art procedures.

5,10-dideazatetrahydrofolic acid is a known antineoplastic agent whose chemical abstract name is (+/-)-N-[4-[2-(2-amino-3,4,5,6,7,8-hexahydro-4-oxopyrido[2,3-d]-pyrimidin-6-yl)ethyl]benzoyl]- L -glutamic acid which has the structure:

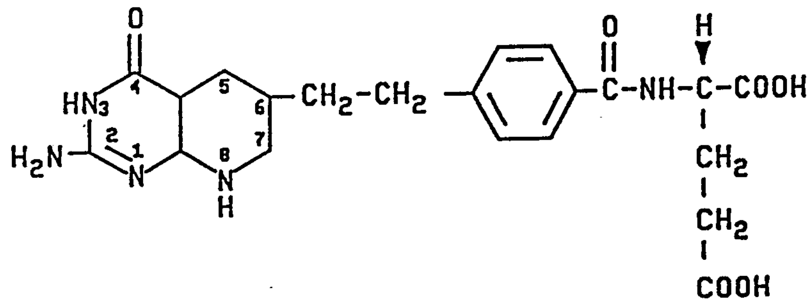

The above compound and the methods for its preparation are disclosed by E.C. Taylor et. al.; (J. Med. Chem. 28, 914 (1985)].

This invention is concerned with a five step process for producing 5,10-dideazatetrahydrofolic acid. This process is illustrated by the following diagrams, which also include the preparation of starting materials that were prepared by known literature procedures. The procedures are described in the text which follows the diagrams wherein advantages over heretofore known methods of producing DDA-THF are discussed thereinafter:

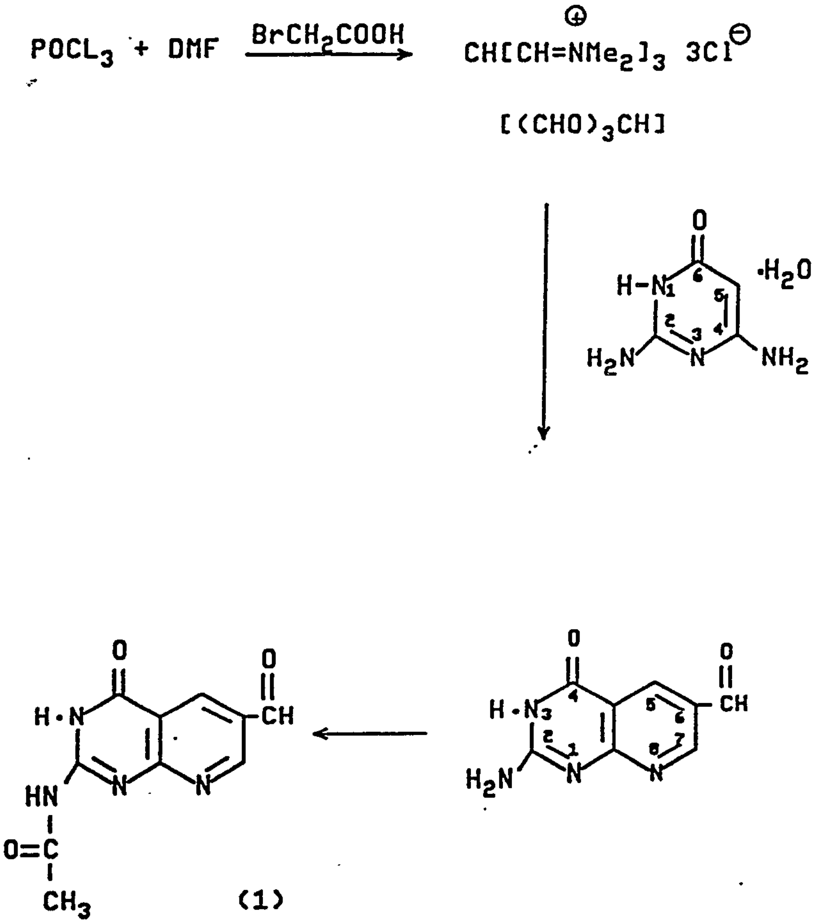


(1)

COMPOUND 2

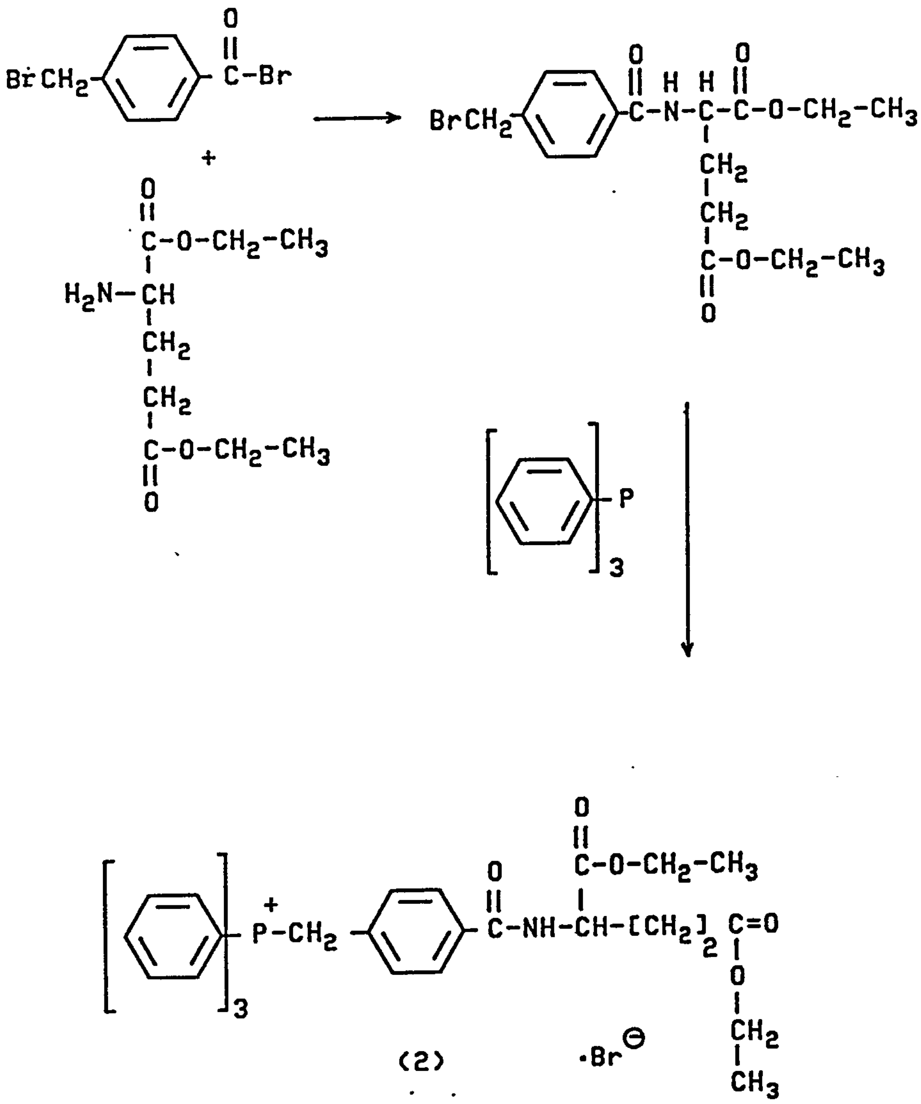

(2)

COMPOUND (1) + COMPOUND (2)

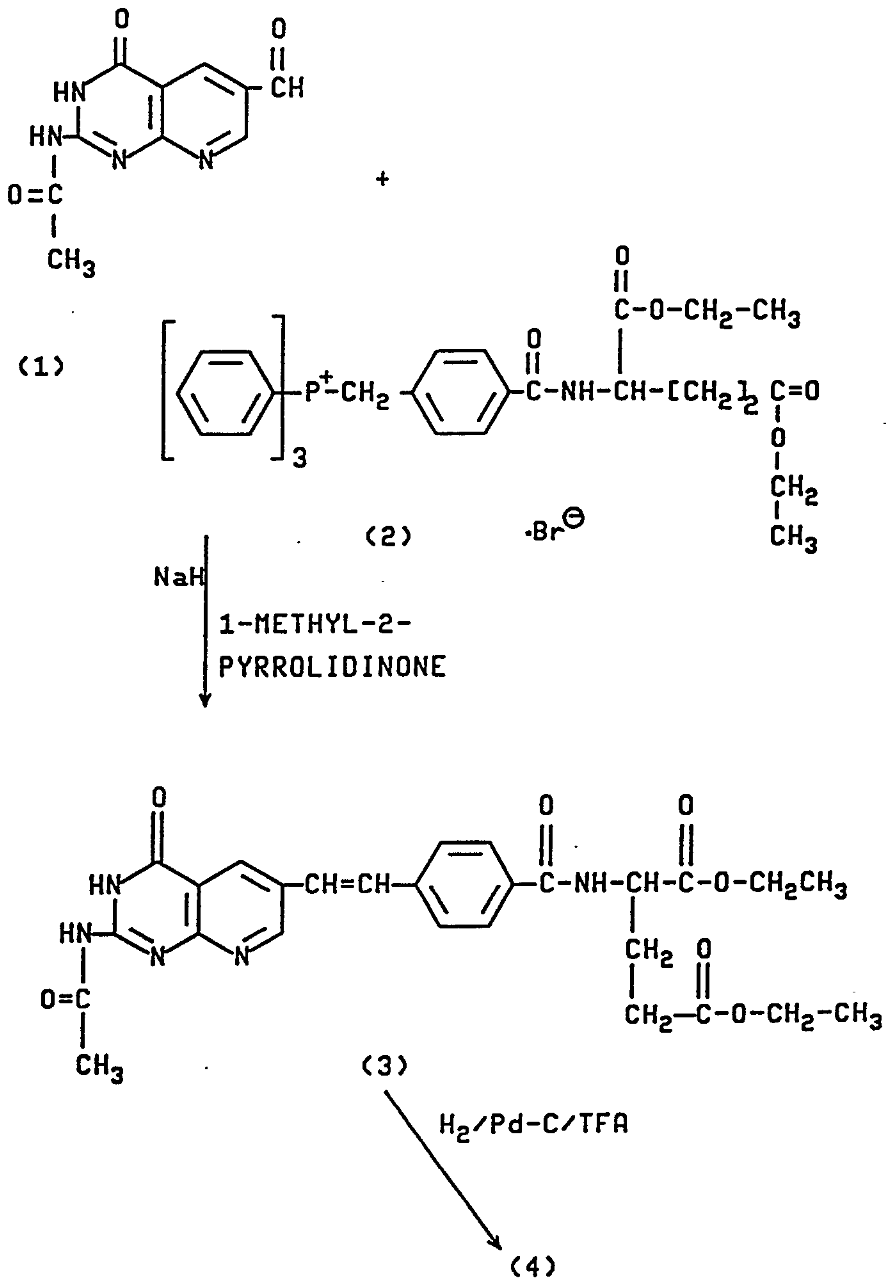


(1)

(2)

(3)

(4)

COMPOUND (1) + COMPOUND (2) (continued)

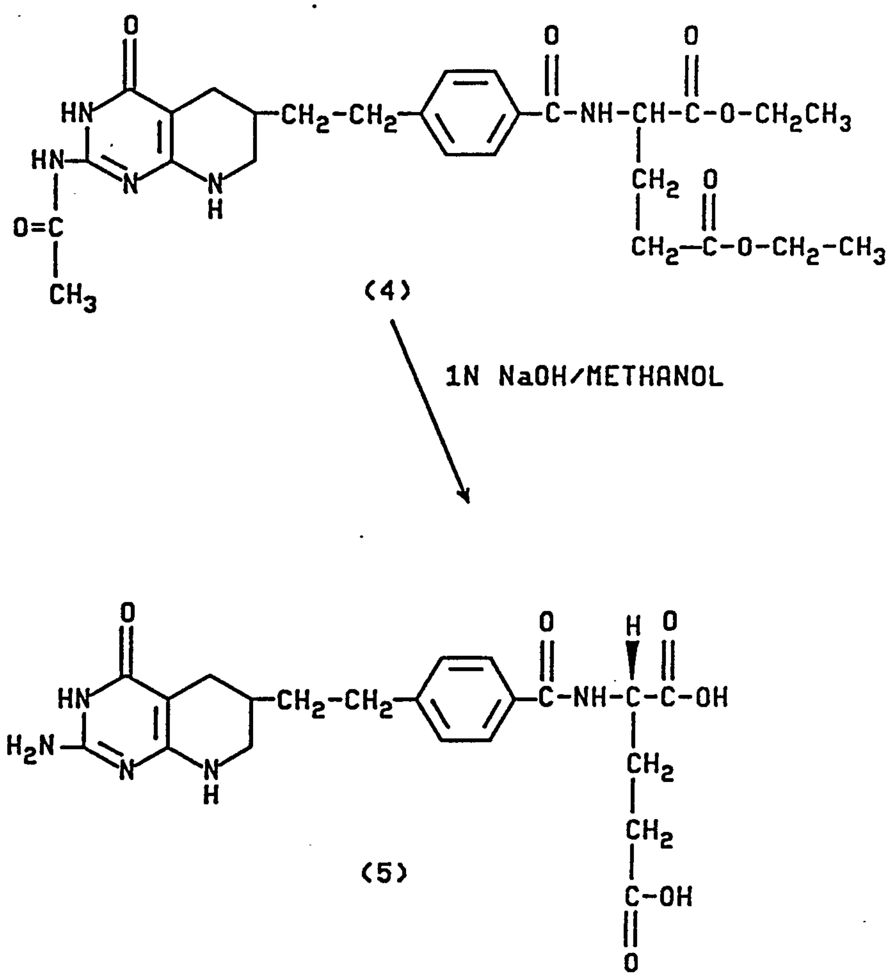


(4)

(5)

Step 1 Synthesis of Compounds (1) and (2)

The compound 2-amino-4(3H)-oxopyrido-[2,3-d]-pyrimidine-6-carboxaldehyde was prepared according to the procedure of C.Temple et al.-[J. Org. Chem. 47, 761(1982)]. These workers found the purification of triformylmethane to be laborious and unnecessary. They used a mixture of the crude intermediate quarternary salt and its partially hydrolyzed derivatives in the condensation with 2,4-diamino-pyrmidin-6(1H)-one. The resultant 5-deazapteridine was acylated by the procedure of E.C. Taylor et al. [J. Org. Chem. 48, 4852 (1983) to give the compound N-(6-formyl-3,4-dihydro-4-oxopyrido[2,3-d]pyr8midin-2-yl) acetamide, compound (1).

The compound [[4-[[[4-ethoxy-1-(ethoxycarbonyl)-4-oxobutyl]amino]carbonyl]phenyl]methyl]triphenylphosphonium bromide, compound (2) was prepared in two steps from p-(bromomethyl)benzoyl bromide according to the procedure of S.J. Yan et al. [J. Hetero Chem. 16, 541(1979)].

Step 2 Coupling of Compound (1) and Compound (2)

The condensation of N-(6-formyl-3,4-dihydro-4-oxopyrido[2,3-d]pyrimidin-2-yl)acetamide, compound (1), with [[4-[[[4-ethoxy-1-(ethoxycarbonyl)-4-oxobutyl]amino]-carbonyl]phenyl]methyl]triphenylphosphonium bromide, compound (2), was carried out at room temperature, under nitrogen using sodium hydride as the base, with 1-methyl-2-pyrrolidinone selected as the solvent. The solvent was dried over 4A molecular sieves prior to use. The above reaction was monitored by work up of an aliquot followed by inspection of the 300 MHz NMR spectrum of this material. In general, about three weeks is required to complete the reaction. For purification purposes, it is best not to use a large excess of phosphonium salt - compound (2). The solvent was removed in vacuo, then a solvent such as toluene or the like is added and the mixture was stirred at room temperature for a short period and was filtered. The recovered solid was dissolved in a minimum amount of a solvent such as N,N-dimethylformamide or the like and purified by flash column chromatography eluting with 10% methanol in ethyl acetate to obtain a 1:1 mixture of cis to trans isomers of N-[4-[2-[2-(acetylamino)-3,-4-dihydro-4-oxo-pyrido[2,3-d]pyrimidin-6-yl]ethenyl]benzoyl]- L-glutamic acid, diethyl ester, compound (3),.in 39% yield.

Step 3 Hydrogenation of Compound (3)

The conversion of [E(andZ)-N-[4-[2-[2-(acetylamino)-3,4-dihydro-4-oxopyrido[2,3-d]pyrmidin-6-yl]-ethenyl]benzoyl]- L-glutamic acid, diethyl ester, compound (3) to N-[4-[2-[2-(acetylamino)-3,4,5,6,7,8-hexahydro-4-oxopyrido[2,3-d]pyrimidin-6-yl]ethyl]benzoyl]- L-glutamic acid, diethyl ester, compound (4) was carried out by hydrogenation at 55 psi for two days, of a solution of compound (3) in trifluoroacetic acid containing 5% palladium on carbon, to provide an 80% yield of compound (4). The conversion of compound (3) to compound (4) is reported by E.C. Taylor et al.; [J. Med. Chem 28, 914(1985) using a reaction time of 14 hours to yield 18% of compound (4) with the majority of product (56%) resulting from the ring oxidized derivative of compound (4).

Step 4 Hydrolysis of Compound (4)

The hydrolysis of N-[4-[2-[2-(acetylamino)-3,-4,5,6,7,8-hexahydro-4-oxopyrido[2,3-d]pyrimidin-6-yl]-ethyl]benzoyl]- L-glutamic acid, diethyl ester, compound (4) in methanol with 1N sodium hydroxide was conducted for 72 hours following the procedure of E.C. Taylor et al. cited hereinabove. Then the volatiles were removed in vacuo and a minimum of water was added. The solu tion was neutralized with 0.5N hydrochloric acid and the solid was collected by filtration, then washed with water, acetone or methanol and finally ether followed by drying to give the desired product 5,10-dideazatetrahydrofolic acid, compound (5) in 82% yield.

Detailed synthesis of [E(and Z)-N-[4-[2-[2-(Acetylamino)-3,4-dihydro-4-oxopyrido)2,3-d]-pyrimidin-6-yl]ethenyl]-benzoyl]-L-glutamic acid, diethyl ester

To a suspension of 101 mg of sodium hydride as a 60% dispersion in oil (2.53 mmol) in 5.0 ml of 1-methyl-2-pyrrolidinone (dried over 4A molecular sieves) was added via cannula a colorless solution of 1.586 g (2.39 mmol) of [[4-[[[4-ethoxy-1-(ethoxycarbonyl)-4-oxobutyl]amino]carbonyl]phenyl]methyl]-triphenylphosphonium bromide in 20 ml of 1-methyl-2-pyrrolidinone. The resultant bright orange solution was stirred at room temperature under nitrogen for 40 minutes, and then added via cannula to 511.3 mg (2.20 mmol) of N-(6-formyl-3,4-dihydro-4-oxopyrido[2,3-d]-pyrimidin-2-yl)acetamide. After 18 days the solvent was removed in vacuo. Toluene was added and the mixture was stirred at room temperature for 20 minutes. The resultant tan solid was collected by filtration to give 1.50 g of crude product.

A 718 mg portion of the above crude product was dissolved in a minimum amount of N,N-dimethylformamide and chromatographed on an 18 x 4.5 cm column of grade 60 silica gel eluting with 10% methanol in ethyl acetate to give 230.8 mg of a bright yellow solid, (A), mp 232-245° C. The 300 MHz NMR spectrum of the material showed it to be a 55 to 45 mixture of cis to trans isomers of the desired product. This ratio was determined by assuming the coupling constant for the trans alkene protons to be 16 Hz and the value of the cis alkene protons to be 12 Hz.

The remainder of the crude product, 784 mg, was chromatographed in the manner described above to give 228.6 mg of a bright yellow solid, (B) mp 225-250 °C. The 300 MHz NMR spectrum of this material showed it to be a 45 to 55 mixture of cis to trans isomers. The total yield (A)+(B) was 459.4 mg of the desired product in 39% yield.

Our convergent synthesis for the preparation of DDA-THF from known aldehyde (1) and phosphonium salt (2) in 3 steps with 12% yield from a commercially available material is a decided improvement over the route reported by E.C. Taylor et al.; [J. Med. Chem. 28. 914 (1985)] that requires 14 steps and provides DDA-THF in only 2% yield.

The synthesis of 5,10-dideazaminopterin which can be used to prepare DDA-THF has been reported by E.C. Taylor et al.; [J. Med. Chem. 28, 914 (1985)] and J. A. Lawson et al.; (J. Hetero Chem. 23, 1 (1986). The procedure of Taylor et al. requires 11 steps and provides only 1% overall yield. Two separate procedures by Lawson et al. require 14 and 9 steps with an overall yield in both cases of less than 1%.

## Claims

1. An improved process for producing (+/-)-N-[4-[2-(2-amino-3,4,5,6,7,8-hexahydro-4-oxopyrido[2,3-d]-pyrimidin-6-yl)ethyl]benzoyl]- L-glutamic acid characterized by:

a) adding a solution of 2.39 mmol of [[4-[[[4-ethoxy-1-(ethoxycarbonyl)-4-oxobutyl]amino]carbonyl]-phenyl]methyl]triphenylphosphonium bromide in dried 1-methyl-2-pyrrolidinone to a suspension of 2.53 mmol of sodium hydride (60% dispersion in oil) in dried 1-methyl-2-pyrrolidinone and stirring the mixture at room temperature, under nitrogen, then

(b) adding the solution to 2.20 mmol of N-(6-formyl-3,4-dihydro-4-oxopyrido[2,3-d-]pyrimidin-2-yl)-acetamide followed by:

c) removing the solvent in vacuo and treating the residue with toluene, then collecting the crude product by filtration;

d) dissolving the crude product in N,N-dimethylformamide and subjecting the solution to column chromatography on grade 60 silica gel and eluting with 10% methanol in ethyl acetate to obtain a 1:1 mixture of cis to trans isomers of N-[4-[2-[2-(acetylamino)-3,4-dihydro-4-oxopyrido[2,3-d]pyrimidin-6-yl]-ethenyl]benzoyl]- L-glutamic acid, diethyl ester, then

e) hydrogenating a mixture of the diethyl ester in trifluoroacetic acid containing 5% palladium on carbon at 55 psi to obtain the compound N-[4-[2-[2-(acetylamino)-3,4,5,6,7,8-hexahydro-4-oxopyrido[2,3-d]-pyrimidin-6-yl]ethyl]benzoyl]- L-glutamic acid diethyl ester, then;

f) subjecting the hexahydro diethyl ester to hydrolysis in methanol with 1N sodium hydroxide, then removing the volatiles in vacuo, adding water, neutralizing the mixture with 0.5N hydrochloric acid and collecting the desired product.